# EUROPEAN PATENT SPECIFICATION

(11) **EP 3 439 631 B1**
(45) Date of publication and mention of the grant of the patent: **10.05.2023**
(21) Application number: 17714281.7
(22) Date of filing: 17.02.2017
(51) Int. Cl.: A61K 33/18

(54) **OPHTHALMIC COMPOSITION COMPRISING PVP-I**
OPHTHALMISCHE ZUSAMMENSETZUNG MIT PVP-I
COMPOSITION OPHTALMIQUE CONTENANT DE LA PVP-I

(30) Priority: 08.04.2016 IT UA20162425
(43) Date of publication of application: 13.02.2019
(73) Proprietor: Medivis S.R.L., 95030 Tremestieri Eneo (CT) (IT)
(72) Inventor: ALEO, Danilo, 95030 Tremestieri Eneo CT (IT); CRO, Melina, 95030 Tremestieri Eneo CT (IT); MANGIAFICO, Sergio, 95030 Tremestieri Eneo CT (IT); MELILLI, Barbara, 95030 Tremestieri Eneo CT (IT); SAITA, Maria Grazia, 95030 Tremestieri Eneo CT (IT)
(74) Representative: Croce, Valeria
(86) International application number: PCT/IB2017/050911
(87) International publication number: WO 2017/175075

(56) References cited:
- WO-A1-2013/078998
- US-A- 5 863 556
- US-A1- 2007 219 170

## Description

The object of the present invention is an ophthalmic composition which comprises Povidone-iodine (PVP-I) at a concentration of between 0.2% - 1.0% (w/w), wherein said ophthalmic composition has an aqueous phase and an oil phase, said oil phase consisting of medium-chain triglycerides and said oil phase constituting less than 3% of the total weight of said composition.

### Prior art

Povidone-iodine (PVP-I) is a complex obtained by combining the polymer polyvinylpyrrolidone (PVP) and iodine which contains on average 10% by weight of available iodine, where for the purposes of the present invention by available iodine it is meant the iodine titratable with thiosulphate, and not more than 6.6% by weight of iodide ion.

PVP-I is widely used as a disinfectant of skin wounds and for oral hygiene.

It is widely confirmed that the bactericidal action of the PVP-I-based drug compositions is to be attributed only to the free molecular iodine I₂ and not to that available titratable with thiosulfate (J. Hickey, J. Pharm. Pharmacol. 1997, 49: 1195-1199; Berkelman R.L., J. Clinical Microbiol. 1982, 635-639).

5% solutions of PVP-I are used for preoperative eye prophylaxis only after local anesthesia, since the instillation causes burning and redness of the eye. It is also reported that galenic ophthalmic preparations at 2.5% PVP-I are very poorly tolerated while those with a PVP-I content of between 1 and 1.25% are better, their instillation in fact only causes a temporary burning.

The need for preparations with a low content of PVP-I is also dictated by the fact that diluted aqueous solutions of PVP-I have a significantly higher antimicrobial activity compared to the same concentrated solutions, since they are able to ensure higher concentrations of free molecular iodine I₂ (Rackur H., Journal of Hospital Infection 1985, 6 Suppl., 13-23). In particular, I₂ appears to be in concentrations of about 1 ppm in 10% solutions of PVP-I and becomes about 20 ppm in solutions with PVP-I concentration of 0.1%. Rackur explains this phenomenon with the presence of PVP polymeric aggregates able to trap I₂, aggregates which in diluted solutions disintegrate, thus releasing said I₂ more easily than in those concentrated (Rackur H., Proc. Intl. Symp. on Povidone 1983, Digenis D.J., Ansell J., eds., University of Kentucky College of Pharmacy, p.99).

In aqueous solution, the maximum concentration of free molecular iodine is reached for concentrations of PVP-I close to 0.1%.

To date, no compositions are available where PVP-I is present at the desired low concentration due to stability problems: the concentration of I₂ over time decreases in a manner inversely proportional to the concentration of PVP-I. Therefore, the more PVP-I is diluted, the more it is unstable.

The free molecular iodine is dispersed as it passes through the walls of the container in which said composition is contained. BASF Pharma Ingredients & Services, Technical Information: PVP-iodine grades August 2010, page 7 describes the influence of the type of packaging material on the stability of an aqueous PVP-I solution at various concentrations, showing that the losses are greater for solutions at a lower concentration of PVP-I and using more permeable materials, such as low-density polyethylene. High density polyethylene has been shown in the same work to have a greater containment action for concentrations of PVP-I above 1%. The containment of high-density polyethylene is ineffective at lower concentrations of PVP-I, for which glass is the most suitable material.

The same BASF Pharma Ingredients & Services, Technical Information: PVP-iodine grades August 2010 on page 10 describes the effect of pH on the stability of PVP-I solutions, showing an increase in the loss of free molecular iodine for PVP-I solutions having a pH greater than 4.5.

Ophthalmic compositions cannot be packaged in a completely sealed glass container, since at least the dropper must be of plastic material, therefore PVP-I solutions for ophthalmic use with concentrations lower than 1%, to date, cannot be packaged and marketed.

Jansen JTG, Povidone-iodine eye drops 0,3% Pharmaceutisch Weekblad 117, 1982 page 420 describes an ophthalmic composition obtained by 30-fold dilution of a 10% PVP-I solution in saline solution. When storing the composition in refrigerated plastic containers, the half-life is 1 month.

US 5,126,127 describes ophthalmic compositions of PVP-I at a concentration of between 0.3 and 0.6% w/w, in the absence of buffer, in a pH range between 2.5 and 4.5. These pH conditions favor the stability of the PVP-I solution (BASF Pharma Ingredients & Services, Technical Information 2010 cited above, page 10) but inevitably make the ophthalmic preparations little tolerated by the human eye.

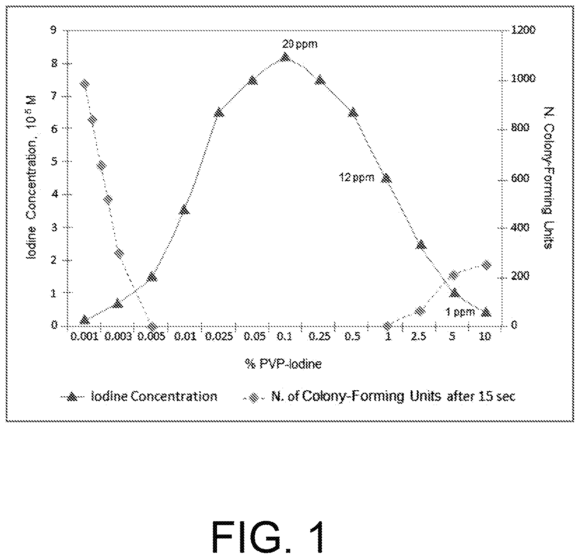

US 5,178,853 describes ophthalmic compositions of PVP-I at a concentration of between 0.3 and 1.0%, at a pH buffered in the range between 5,5 - 6,5, further comprising potassium iodide in amounts greater than 10% by weight with respect to the % of PVP-I. The compositions are stable in PET (polyethylene terephthalate) plastic containers. Said compositions are stable at room temperature for 24 months. Due to the presence of iodide, stable PVP-I formulations are obtained at pH closer to neutral, therefore better tolerated by the ocular tissues. However, iodide (I⁻) has a negative effect on the concentration of free molecular iodine (I₂). I- reacts with I₂ leading to the species triiodide (I₃⁻) which remains in solution without escaping from the container, as the easily volatile species I₂ would. As expected, I₂ measured in said composition is only 2 ppm whereas, in the absence of I⁻, about 20 ppm of I₂ would be expected (see Figure 1).

With similar results, also EP0371283 stabilizes a PVP-I ophthalmic composition with potassium iodide. The proposed solutions are not satisfactory, since a lower concentration of I₂ reduces the germicidal activity of the composition itself.

US 5,863,556 describes compositions for ophthalmic use which comprise PVP-I at a concentration ranging between 0.2 and 1.0% w/w. The peculiarity of the formulation is in that PVP-I is delivered in liposomes. This peculiarity, advantageous for stabilizing PVP-I, however makes the preparation not formulatable in a liquid form for ophthalmic use, where the solid liposomes containing PVP-I would be retained during the filtration necessary for the sterilization of a liquid formulation for ophthalmic use. Other sterilization methods, for example by irradiation or under pressure at high temperature, are not practicable as they would degrade PVP-I.

WO2013/078998 also describes formulations with a low concentration of PVP-I that are suspensions, comprising PVP-I previously co-precipitated with sodium alginate and calcium chloride.

US 2007/219170 describes a solution of PVP-I with an antiinflammatory agent.

While in other areas, for example in the treatment of skin wounds, PVP-I composition at high concentrations, even around 10%, and thus stable, are used successfully and are well tolerated, the need is strongly felt to have a diluted ophthalmic composition of PVP-I, at a concentration lower than 1%, which is well tolerated, sufficiently stable and which maintains significant levels of I₂ over time, able to carry the germicidal activity required, so as to allow its use also in ophthalmic therapy of a disinfectant with proven effectiveness, as is PVP-I (Margreet Hogeweg, Letters to the Editor, Community Eye Health 2003 Vol. 16 No. 48: 63).

### Description

The object of the present invention is an ophthalmic composition which comprises PVP-I at a concentration of between 0.2% - 1.0% (w/w), wherein said ophthalmic composition has an aqueous phase and an oil phase, said oil phase consisting of medium-chain triglycerides and said oil phase constituting less than 3% of the total weight of said composition.

In a further embodiment, said ophthalmic composition further comprises a surfactant and iodide at a concentration of between 0.01% and 1.0% w/w.

### Description of the figures

Figure 1: correlation between the content of free molecular iodine I₂ and the microbial reduction after 15 seconds from treatment with aqueous solutions of PVP-I at different concentrations.

### Detailed description

As shown by the curves shown in Figure 1, the antimicrobial activity of compositions comprising PVP-I is maximum the higher the levels of I₂ and the levels of I₂ are maximum at PVP-I concentrations of between 0.01 and 1%, notoriously unstable concentrations, as discussed in the preceding sections.

The present invention has surprisingly shown that compositions of PVP-I at a concentration of between 0.2% - 1.0%, where said compositions comprise an aqueous phase and an oil phase, said oil phase consisting of medium-chain triglycerides, and said oil phase constituting less than 3% by weight of the total of said composition, keep the levels of I₂ constant and high over time.

Where it is widely known that the presence of iodides negatively affects the availability of free molecular iodine I₂, surprisingly it has also been demonstrated herein that by adding to the composition described herein, in the presence of a surfactant, an iodide in a concentration of between 0.01% and 1.0% w/w, I₂ is kept unexpectedly at significantly high levels, while the prior art suggests values of I₂ of a few ppm in the presence of iodides. This further embodiment is exemplified in the following Examples 15-17. The ophthalmic preparation of the present invention is a composition which comprises an aqueous phase, an oil phase, a surfactant and PVP-I in an amount of between 0.2 and 1.0% w/w, preferably between 0.4 and 0.8% w/w. In said composition, the content of free molecular iodine I₂ at T₀ (freshly prepared) is of between 10 and 100 ppm.

In a further embodiment, said ophthalmic composition further comprises an iodide, preferably potassium iodide or sodium iodide, at a concentration of between 0.01% and 1.0% w/w. In this embodiment, the surfactant is preferably selected from Macrogol (15)-hydroxystearate (SOLUTOL HS 15) and D-alpha tocopheryl polyethylene glycol 1000 succinate (Vitamin E TPGS). Said oil phase is composed of triglycerides, preferably medium-chain triglycerides (MCT), having an aliphatic chain composed of a number of carbon atoms of between 6 and 12. Said MCT are preferably one or more of caproic acid, caprylic acid, capric acid and lauric acid. Said MCT are present in an amount of less than 3% by weight of the total formulation, preferably between 0.05 and 1% w/w, even more preferably between 0.05 and 0.9% w/w. In a preferred embodiment, said MCT are present in amounts of between 0.1 and 0.9% w/w. In the presence of iodides, said oil phase preferably comprises caprylic capric triglyceride. Said surfactant is present in amounts of between 0.05 and 4% w/w, preferably between 0.2 and 2.0% w/w. Preferably, said surfactant is TPGS.

In a further embodiment, said composition is buffered, preferably with phosphate or citrate buffer, at a pH range of between 4.5 and 6.5, preferably between pH 5.4 and 5.9. Alternatively, a strong base can be used for pH adjustment.

Said composition, in a further embodiment, comprises osmotizing agents, preferably selected from glycerol and sodium chloride.

Said composition, in a further embodiment, comprises viscosizing polymers. Among these, preferred is sodium hyaluronate in concentrations of between 0.01 and 0.2% w/w, preferably between 0.04 and 0.08% w/w. In further embodiments, polymers compatible with the ophthalmic application are added, for example selected from carbopol, polyvinyl alcohol and hydroxypropyl cellulose.

The composition according to the present invention may be contained in single-dose and/or multi-dose polyethylene or polypropylene containers of suitable thickness and suitable density. In a preferred embodiment, the composition of the present invention is packaged in glass containers, preferably in amber glass bottles having a dropper, where said dropper is preferably made of polypropylene, polyethylene or bromobutyl rubber. Said bottles are preferably 10 mL bottles.

The composition of the present invention based on PVP-I surprisingly offers the combined advantage of being well tolerated for eye treatments and of having a high content of free molecular iodine, where the chemical-physical stability of the preparation and the levels of free molecular iodine are maintained even long-term. In fact, the formulation according to the present invention, without resorting to addition of iodide with the drawbacks that said addition entails, keeps the titer of PVP-I between 85 and 120% of its nominal titer, according to the USP (*US Pharmacopeial Convention*) for solutions based on PVP-I.

The composition according to the present invention may be administered per se or in the form of a pharmaceutical preparation in which said composition is in a compound or mixture with one or more pharmaceutically acceptable excipients. Pharmaceutical preparations for use according to the present invention may be formulated in a conventional manner using one or more pharmaceutically acceptable excipients including carriers, diluents and adjuvants. In a further embodiment, said pharmaceutical preparation advantageously comprises, in addition to the composition according to the present invention, one or more further active ingredients. Preferably, said pharmaceutical preparation is in the form of an eye drop.

A further aspect of the present invention is a method for treating ophthalmic pathologies comprising administering to a subject in need thereof the formulation according to the present invention.

The invention is illustrated in the following Examples which are intended to better describe it, without limiting the scope thereof.

### Examples

Formulations tested in the Examples:
Examples 1, 2, 3: compositions comprising PVP-I at different concentrations, MCT 0.11% w/w, TPGS 0.39% w/w (Table 1)
Examples 4, 5, 6: compositions comprising PVP-I 0.66% w/w, and different concentrations of MCT and TPGS (Table 2)
Examples 7, 8, 9: compositions also comprising phosphate buffer (Table 3)
Examples 10, 11, 12: compositions also comprising polymers (Table 4)
(Comparative) Examples 13, 14: compositions comprising MCT at concentrations equal to 3% and 8% w/w (Table 5)
Examples 15, 16, 17: compositions also comprising potassium iodide (Table 6).

**Table 1**

| **Ingredients** | Example 1 % w/w | Example 2 % w/w | Example 3 % w/w |
|---|---|---|---|
| PVP-I 10% | 0.66 | 0.4 | 1.0 |
| Caprylic capric triglyceride | 0.11 | 0.11 | 0.11 |
| TPGS | 0.39 | 0.39 | 0.39 |
| Potassium citrate tribasic monohydrate | 0.30 | 0.30 | 0.30 |
| Citric acid monohydrate | 0.025 | 0.025 | 0.025 |
| Glycerol | 0.90 | 0.90 | 0.90 |
| Sodium hyaluronate | 0.05 | 0.05 | 0.05 |
| Sodium chloride | 0.45 | 0.45 | 0.45 |
| Purified water | Up to 100 g | Up to 100 g | Up to 100 g |
| | | | |
| Free iodine I₂ | 22 ppm | 33 ppm | 17 ppm |

**Table 2**

| **Ingredients** | Example 4 % w/w | Example 5 % w/w | Example 6 % w/w |
|---|---|---|---|
| PVP-I 10% | 0.66 | 0.66 | 0.66 |
| Caprylic capric triglyceride | 0.5 | 0.8 | 1.0 |
| TPGS | 1.0 | 2.0 | 1.0 |
| Potassium citrate tribasic monohydrate | 0.30 | 0.30 | 0.30 |
| Citric acid monohydrate | 0.025 | 0.025 | 0.025 |
| Glycerol | 0.90 | 0.90 | 0.90 |
| Sodium hyaluronate | 0.05 | 0.05 | 0.05 |
| Sodium chloride | 0.45 | 0.45 | 0.45 |
| Purified water | Up to 100 g | Up to 100 g | Up to 100 g |
| | | | |
| Free iodine I₂ | 62 ppm | 78 ppm | 66 ppm |

**Table 3**

| **Ingredients** | Example 7 % w/w | Example 8 % w/w | Example 9 % w/w |
|---|---|---|---|
| PVP-I 10% | 0.66 | 0.66 | 0.66 |
| Caprylic capric triglyceride | 0.11 | 0.11 | 1.0 |
| TPGS | 0.39 | 0.39 | 1.0 |
| Disodium hydrogen phosphate dodecahydrate | 0.25 | 0.25 | 0.25 |
| Sodium dihydrogen phosphate dihydrate | 0.05 | 0.05 | 0.05 |
| Glycerol | 0.90 | 0.90 | 0.90 |
| Sodium hyaluronate | 0.05 | 0.08 | 0.2 |
| Sodium chloride | 0.45 | 0.45 | 0.45 |
| Purified water | Up to 100 g | Up to 100 g | Up to 100 g |
| | | | |
| Free iodine I₂ | 14 ppm | 16 ppm | 55 ppm |

**Table 4**

| **Ingredients** | Example 10 % w/w | Example 11 % w/w | Example 12 % w/w |
|---|---|---|---|
| PVP-I 10% | 0.66 | 0.66 | 0.66 |
| Caprylic capric triglyceride | 0.11 | 0.11 | 0.11 |
| TPGS | 0.39 | 0.39 | 0.39 |
| Potassium citrate tribasic monohydrate | 0.30 | - | 0.30 |
| Citric acid monohydrate | 0,025 | - | 0, 025 |
| Glycerol | 0.90 | 1.80 | 0.90 |
| Hydroxymethyl propylcellulose | 0.4 | | |
| Carbopol | | 0.2 | |
| Polyvinyl alcohol | | | 0.5 |
| Sodium chloride | 0.45 | - | 0.45 |
| Purified water | Up to 100 g | Up to 100 g | Up to 100 g |
| | | | |
| Free iodine I₂ | 19 ppm | 23 ppm | 22 ppm |

**Table 5**

| **Ingredients** | Example 13 % w/w | Example 14 % w/w |
|---|---|---|
| PVP-I 10% | 1.0 | 1.0 |
| Caprylic capric triglyceride | 3.0 | 8.0 |
| TPGS | 1.5 | 12.0 |
| Potassium citrate tribasic monohydrate | 0.30 | 0.30 |
| Citric acid monohydrate | 0.025 | 0.025 |
| Glycerol | 0.90 | 0.90 |
| Sodium hyaluronate | 0.05 | 0.05 |
| Sodium chloride | 0.45 | 0.45 |
| Purified water | Up to 100 g | Up to 100 g |
| | | |
| Free iodine I₂ | 120 ppm | 195 ppm |

**Table 6**

| **Ingredients** | Example 15 % w/w | Example 16 % w/w | Example 17 % w/w |
|---|---|---|---|
| PVP-I 10% | 0.25 | 0.4 | 1.0 |
| Caprylic capric triglyceride | 0.60 | 0.80 | 0.80 |
| SOLUTOL HS15 | 2.4 | 2.6 | 2.6 |
| Potassium iodide | 0.05 | 0.20 | 1.0 |
| Potassium citrate tribasic monohydrate | 0.30 | 0.30 | 0.30 |
| Citric acid monohydrate | 0.025 | 0.025 | 0.025 |
| Glycerol | 0.90 | 0.90 | 0.90 |
| Sodium hyaluronate | 0.05 | 0.05 | 0.05 |
| Sodium chloride | 0.45 | 0.45 | 0.45 |
| Purified water | Up to 100 g | Up to 100 g | Up to 100 g |
| | | | |
| Free iodine I₂ | 31 ppm | 28 ppm | 25 ppm |

The compositions according to Examples 1-17 were packaged in 10 mL amber glass vials, provided with polyethylene dropper.

The 24-month stability at 4°C was obtained by measuring the PVP-I levels, finding levels always higher than 85% of the starting PVP-I levels.

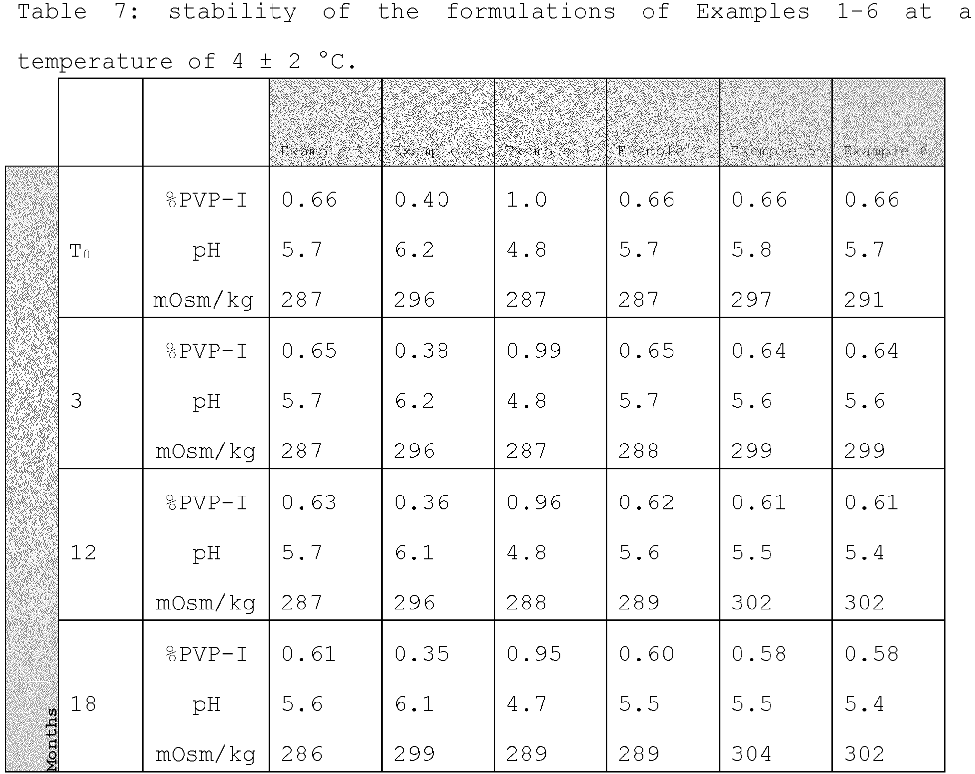

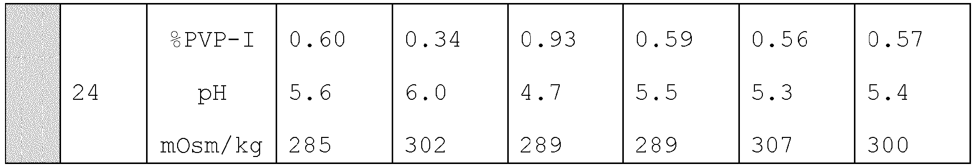

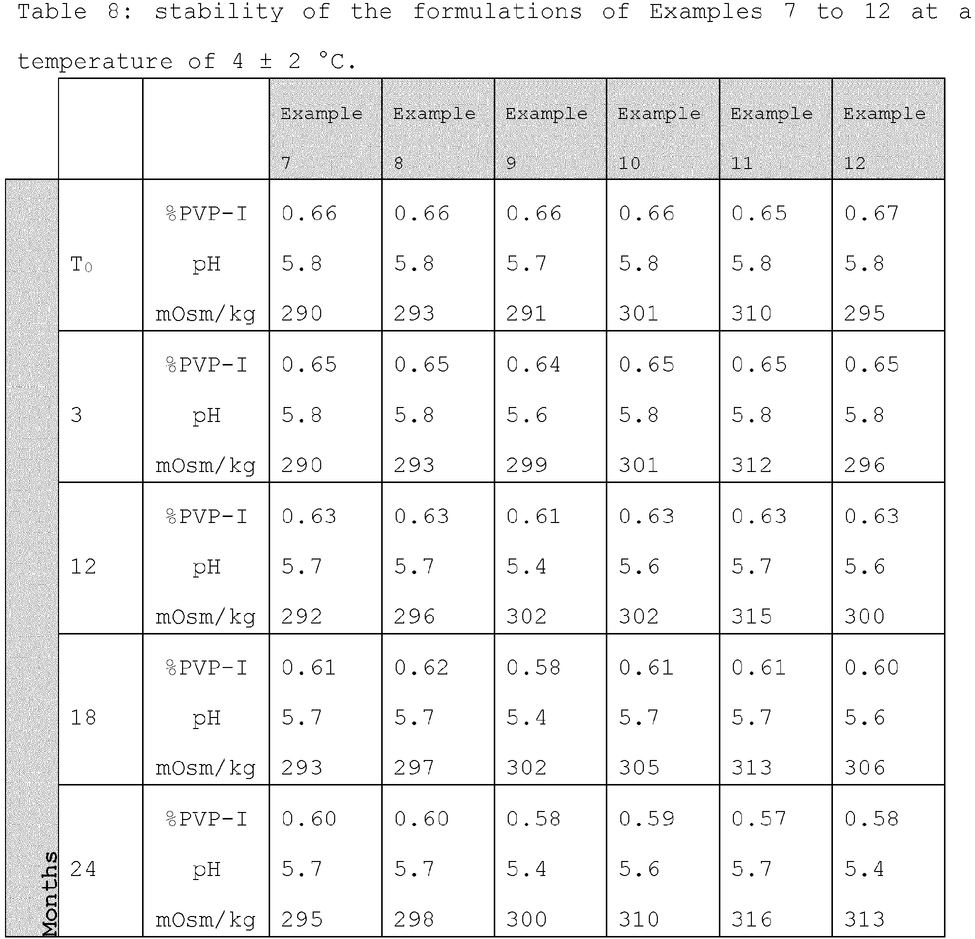

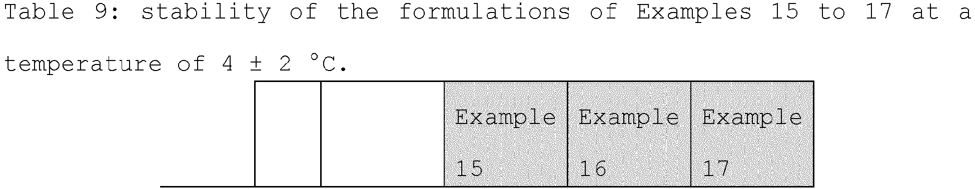

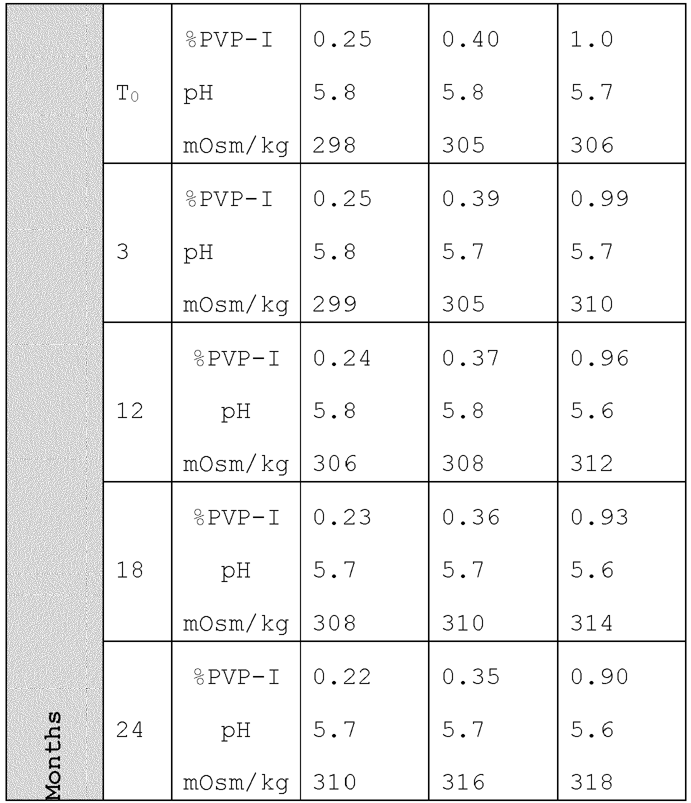

For each of the formulations tested in Examples 1 to 12 and 15 to 17, the values of PVP-I at 24 months are always above 85% with respect to the free molecular iodine at T₀.

Table 10 shows, by way of comparison, the stability data obtained with the formulations of Examples 13 and 14, where there is an excess of MCT.

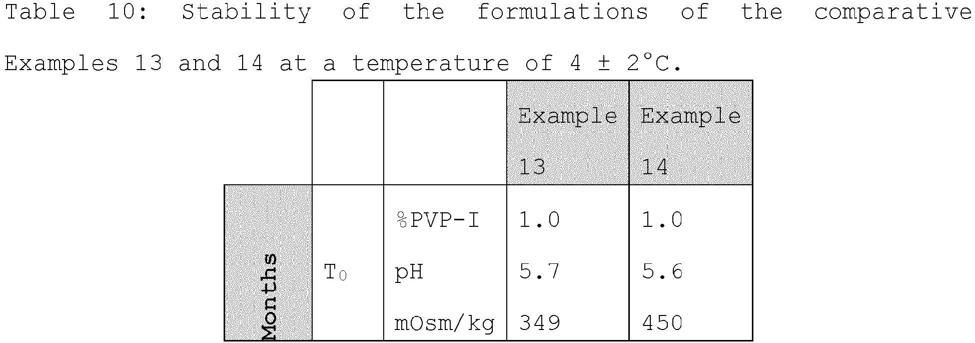

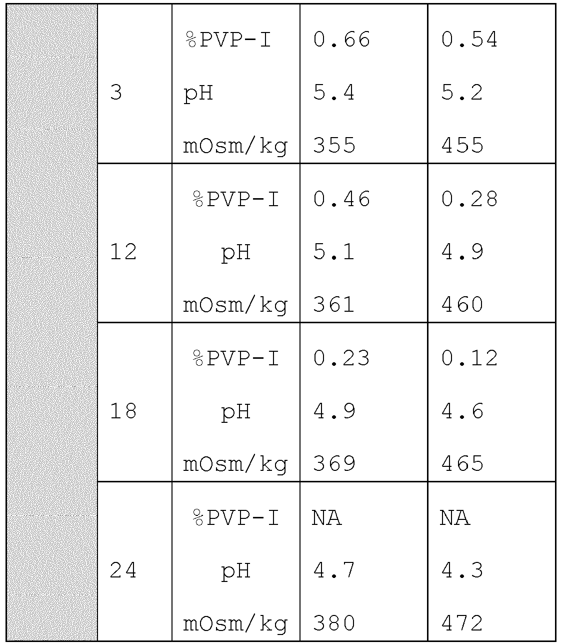

The chemical-physical stability of the formulation is greatly compromised, as evidenced by the pH and osmolarity data. Moreover, at 18 months the PVP-I in the formulation of Example 13 drops to 23% of the starting value, in the formulation of 14 to 12% of the starting value. At 24 months, no measurable PVP-I is left in the composition.

## Claims

1. An ophthalmic composition comprising an aqueous phase, an oil phase, and Povidone-iodine (PVP-I) at a concentration of between 0.2% - 1.0% (w/w), said oil phase consisting of medium-chain triglycerides and said oil phase constituting less than 3% of the total weight of said composition.

2. The ophthalmic composition according to claim 1, wherein said PVP-I is at a concentration of between 0.4 and 0.8% w/w.

3. The ophthalmic composition according to one of claims 1 or 2, wherein said triglycerides are selected from one or more of caproic acid, caprylic acid, capric acid and lauric acid, preferably it is caprylic capric triglyceride.

4. The ophthalmic composition according to one of claims 1 to 3, wherein said triglycerides are present in amounts of between 0.05 and 1% w/w, preferably between 0.05 and 0.9% w/w, or between 0.1 and 0.9% w/w.

5. The ophthalmic composition according to one of claims 1 to 4, further comprising a surfactant, wherein said surfactant is in amounts of between 0.05 and 4% w/w, preferably between 0.2 and 2.0% w/w and preferably said surfactant is d-α-tocopheryl polyethylene glycol 1000 succinate (TPGS).

6. The ophthalmic composition according to claim 5, further comprising an iodide in amounts of between 0.01 and 1.0% w/w, wherein said iodide is preferably potassium iodide or sodium iodide.

7. The ophthalmic composition according to one of claims 1 to 6, which is buffered, preferably with phosphate or citrate buffer, in a pH range of between 4.5 and 6.5, preferably between pH 5.4 and 5.9.

8. The ophthalmic composition according to one of claims 1 to 7, further comprising osmotizing agents, preferably selected from glycerol and sodium chloride and/or viscosizers, preferably sodium hyaluronate.

9. The ophthalmic composition according to one of claims 1 to 8, comprising:
- PVP-I 0.4-0.8% w/w
- Medium chain triglycerides 0.1-0.9% w/w
- TPGS 0.2 - 2.0% w/w
- Water as needed
having a pH of between 4.5 and 6.5.

10. The ophthalmic composition according to one of claims 1 to 8, comprising:
- PVP-I 0.25-1.0% w/w
- Caprylic capric triglyceride 0.60-0.80% w/w
- SOLUTOL HS15 2.4 - 2.6% w/w
- Potassium iodide 0.05-1.0% w/w
- Water as needed
having a pH of between 4.5 and 6.5.

11. The ophthalmic composition according to one of claims 1 to 10, which is contained in amber glass bottles having a dropper, where said dropper is preferably made of polypropylene, polyethylene or bromobutyl rubber.

12. A pharmaceutical preparation comprising the ophthalmic composition according to one of claims 1 to 10 for use in ophthalmic therapy.

## Patentansprüche

1. Ophthalmische Zusammensetzung, umfassend eine wässrige Phase, eine Ölphase und Povidon-lod (PVP-I) in einer Konzentration zwischen 0,2 Gew.- % - 1,0 Gew.-%, wobei die Ölphase aus mittelkettigen Triglyceriden besteht und die Ölphase weniger als 3 % des Gesamtgewichts der Zusammensetzung ausmacht.

2. Ophthalmische Zusammensetzung nach Anspruch 1, wobei das PVP-I in einer Konzentration zwischen 0,4 und 0,8 Gew.-% vorliegt.

3. Ophthalmische Zusammensetzung nach einem der Ansprüche 1 oder 2, wobei die Triglyceride ausgewählt sind aus einem oder mehreren von Capronsäure, Caprylsäure, Caprinsäure und Laurinsäure, wobei es bevorzugt Capryl-Caprin-Triglycerid ist.

4. Ophthalmische Zusammensetzung nach einem der Ansprüche 1 bis 3, wobei die Triglyceride in Mengen zwischen 0,05 und 1 Gew.-%, bevorzugt zwischen 0,05 und 0,9 Gew.-%, oder zwischen 0,1 und 0,9 Gew.-%, vorliegen.

5. Ophthalmische Zusammensetzung nach einem der Ansprüche 1 bis 4, ferner umfassend ein Tensid, wobei das Tensid in Mengen zwischen 0,05 und 4 Gew.-%, bevorzugt zwischen 0,2 und 2,0 Gew.-%, vorliegt und das Tensid bevorzugt d-α-Tocopherylpolyethylenglykol-1000-succinat (TPGS) ist.

6. Ophthalmische Zusammensetzung nach Anspruch 5, ferner umfassend ein lodid in Mengen zwischen 0,01 und 1,0 Gew.-%, wobei das lodid bevorzugt Kaliumiodid oder Natriumiodid ist.

7. Ophthalmische Zusammensetzung nach einem der Ansprüche 1 bis 6, die gepuffert ist, bevorzugt mit Phosphat- oder Citratpuffer, in einem pH-Bereich zwischen 4,5 und 6,5, bevorzugt zwischen pH 5,4 und 5,9.

8. Ophthalmische Zusammensetzung nach einem der Ansprüche 1 bis 7, ferner umfassend Osmotisierungsmittel, bevorzugt ausgewählt aus Glycerin und Natriumchlorid, und/oder Viskositätsregler, bevorzugt Natriumhyaluronat.

9. Ophthalmische Zusammensetzung nach einem der Ansprüche 1 bis 8, umfassend:
- PVP-I 0,4-0,8 Gew.-%
- Mittelkettige Triglyceride 0,1-0,9 Gew.-%
- TPGS 0,2 - 2,0 Gew.-%
- Wasser nach Bedarf
mit einem pH-Wert zwischen 4,5 und 6,5.

10. Ophthalmische Zusammensetzung nach einem der Ansprüche 1 bis 8, umfassend:
- PVP-I 0,25-1,0 Gew.-%
- Capryl-Caprin-Triglycerid 0,60-0,80 Gew.-%
- SOLUTOL HS15 2,4 - 2,6 Gew.-%
- Kaliumiodid 0,05-1,0 Gew.-%
- Wasser nach Bedarf
mit einem pH-Wert zwischen 4,5 und 6,5.

11. Ophthalmische Zusammensetzung nach einem der Ansprüche 1 bis 10, die in Braunglasflaschen mit einer Pipette enthalten ist, wobei die Pipette bevorzugt aus Polypropylen, Polyethylen oder Brombutylkautschuk hergestellt ist.

12. Pharmazeutische Zubereitung, umfassend die ophthalmische Zusammensetzung nach einem der Ansprüche 1 bis 10 zur Verwendung in der ophthalmischen Therapie.

## Revendications

1. Composition ophtalmique comprenant une phase aqueuse, une phase huileuse et de la povidone iodée (PVP-I) selon une concentration comprise entre 0,2 % et 1,0 % (en poids), ladite phase huileuse étant constituée de triglycérides à chaîne moyenne et ladite phase huileuse constituant moins de 3 % du poids total de ladite composition.

2. Composition ophtalmique selon la revendication 1, dans laquelle ladite PVP-1 est présente selon une concentration comprise entre 0,4 et 0,8 % en poids.

3. Composition ophtalmique selon l'une des revendications 1 ou 2, dans laquelle lesdits triglycérides sont choisis parmi un ou plusieurs des composants acide caproïque, acide caprylique, acide caprique et acide laurique, et il s'agit de préférence de triglycérides capryliques / capriques.

4. Composition ophtalmique selon l'une des revendications 1 à 3, dans laquelle lesdits triglycérides sont présents en quantités comprises entre 0,05 et 1 % en poids, de préférence entre 0,05 et 0,9 % en poids, ou entre 0,1 et 0,9 % en poids.

5. Composition ophtalmique selon l'une des revendications 1 à 4, comprenant en outre un tensioactif, dans laquelle ledit tensioactif est présent en quantités comprises entre 0,05 et 4 % en poids, de préférence entre 0,2 et 2,0 % en poids, et dans laquelle ledit tensioactif est de préférence le succinate de d-α-tocophéryl-polyéthylèneglycol 1000 (TPGS).

6. Composition ophtalmique selon la revendication 5, comprenant en outre un iodure en quantités comprises entre 0,01 et 1,0 % en poids, dans laquelle ledit iodure est de préférence un iodure de potassium ou un iodure de sodium.

7. Composition ophtalmique selon l'une des revendications 1 à 6, qui est tamponnée, de préférence avec un tampon phosphate ou citrate, dans une plage de pH comprise entre 4,5 et 6,5, de préférence dans une plage de pH entre 5,4 et 5,9.

8. Composition ophtalmique selon l'une des revendications 1 à 7, comprenant en outre des agents osmoseurs, de préférence choisis parmi le glycérol et le chlorure de sodium et/ou des agents de viscosité, de préférence le hyaluronate de sodium.

9. Composition ophtalmique selon l'une des revendications 1 à 8, comprenant :
- de la PVP-I à 0,4 à 0,8 % en poids
- des triglycérides à chaîne moyenne à 0,1 à 0,9 % en poids
- du TPGS à 0,2 à 2,0% en poids
- de l'eau tel que nécessaire,
et ayant un pH compris entre 4,5 et 6,5.

10. Composition ophtalmique selon l'une des revendications 1 à 8, comprenant :
- de la PVP-1 à 0,25 à 1,0 % en poids
- des triglycérides capryliques / capriques à 0,60 à 0,80 % en poids
- du SOLUTOL HS15 à 2,4 à 2,6 % en poids
- du iodure de potassium à 0,05 à 1,0 % en poids
- de l'eau tel que nécessaire,
et ayant un pH compris entre 4,5 et 6,5.

11. Composition ophtalmique selon l'une des revendications 1 à 10, qui est contenue dans des flacons en verre ambré munis d'un compte-gouttes, ledit compte-gouttes étant de préférence en polypropylène, en polyéthylène ou en caoutchouc de bromobutyle.

12. Préparation pharmaceutique comprenant la composition ophtalmique selon l'une des revendications 1 à 10 pour une utilisation en thérapie ophtalmique.
